# EUROPEAN PATENT SPECIFICATION

(11) **EP 2 505 150 B1**
(45) Date of publication and mention of the grant of the patent: **06.11.2019**
(21) Application number: 12162601.4
(22) Date of filing: 30.03.2012
(51) Int. Cl.: A61B 17/12

(54) **Occlusive device with porous structure and stretch resistant member**
Okklusionsvorrichtung mit poröser Struktur und streckbeständigem Element
Dispositif occlusif avec structure poreuse et élément résistant à l'étirement

(30) Priority: 31.03.2011 US 201113076491
(43) Date of publication of application: 03.10.2012
(73) Proprietor: Codman & Shurtleff, Inc., Raynham, MA 02767 (US)
(72) Inventor: Lorenzo, Juan, Raynham, MA Massachusetts 02767 (US); Forsythe, Peter, Raynham, MA Massachusetts 02767 (US)
(74) Representative: Carpmaels & Ransford LLP

(56) References cited:
- EP-A1- 1 584 298
- WO-A2-2005/039664
- US-A1- 2005 090 861
- US-A1- 2006 058 834
- US-A1- 2006 178 696
- US-A1- 2006 271 086

## Description

### BACKGROUND OF THE INVENTION

### 1. Field of the Invention

The invention relates to implants within body vessels and more particularly to occlusive devices including embolic coils having resistance to stretching.

### 2. Description of the Related Art

Vascular disorders and defects such as aneurysms and other arterio-venous malformations are especially difficult to treat when located near critical tissues or where ready access to a malformation is not available. Both difficulty factors apply especially to cranial aneurysms. Due to the sensitive brain tissue surrounding cranial blood vessels and the restricted access, it is very challenging and often risky to surgically treat defects of the cranial vasculature.

Alternative treatments include vascular occlusion devices such as embolic coils deployed using catheter delivery systems. In a currently preferred procedure to treat a cranial aneurysm, the distal end of an embolic coil delivery catheter is inserted into non-cranial vasculature of a patient, typically through a femoral artery in the groin, and guided to a predetermined delivery site within the cranium. A number of delivery techniques for vaso-occlusive devices, including use of fluid pressure to release an embolic coil once it is properly positioned, are described by Diaz et al. in U.S. Patent Nos. 6,063,100 and 6,179,857, for example.

Multiple embolic coils of various lengths, commonly 1 to 30 centimetres, and preselected stiffness often are packed sequentially within a cranial aneurysm to limit blood flow therein and to encourage embolism formation. Typically, physicians first utilize stiffer coils to establish a framework within the aneurysm and then select more flexible coils to fill spaces within the framework. Ideally, each coil conforms both to the aneurysm and to previously implanted coils. Each successive coil is selected individually based on factors including stiffness, length, and preformed shape which the coil will tend to assume after delivery.

During implantation, the physician manipulates each embolic coil until it is in a satisfactory position, as seen by an imaging technique such as fluoroscopic visualization, before detaching the coil from the delivery system. It is highly desired for both ends of each coil to remain positioned within the aneurysm after delivery, because a length of coil protruding into the main lumen of the blood vessel invites undesired clotting external to the aneurysm. After each successive coil is detached, the next coil is at an increasing risk of becoming entangled in the growing mass of coils, thereby restricting the depth of insertion for that coil into the aneurysm.

Difficulties may arise due to stretching of the embolic coils during repositioning or attempted retrieval of the coils, especially if the coil becomes entangled and complete insertion of the coil into the aneurysm is not accomplished. If pulling forces applied to a coil exceed its elastic limit, the coil will not return to its original shape. A stretched coil exhibits diminished pushability or retractability, and becomes more difficult to manipulate into an optimal position or to be removed. Moreover, a stretched coil occupies less volume than an unstretched coil, which increases the number of coils needed to sufficiently pack the aneurysm to encourage formation of a robust embolus positioned wholly within the aneurysm.

There have been a number of attempts to address stretch-related problems in embolic coils. Several stretch-resistant devices are disclosed in U.S. Patent No. 5,853,418 to Ken et al., having a primary coil and an elongated stretch-resisting member fixedly attached to the primary coil in at least two locations. While Ken et al. mention possible hydraulic delivery of their coils through a lumen of a catheter, they teach that it is desirable to controllably release each coil using a severable or mechanical joint such as an electrolytically detachable joint. Such joints are not compatible with certain delivery systems, and some physicians prefer not to use electrical currents to detach embolic coils from a delivery catheter.

Another embolic device, described in U.S. Patent No. 6,183,491 by Lulo, has a support wire attached at one end to a proximal end of the coil and attached at its other end to an attachment point located in an intermediate portion of the coil. The embolic device has a closed proximal end and is suitable for hydraulic release from a delivery system after the device is properly positioned. However, only the proximal portion of the coil resists stretching; any length of coil distal to the intermediate attachment point is unprotected from excessive elongation forces.

A vaso-occlusive device described by Schaefer et al. in U.S. Patent Publication No. 2004/0006362 has a number of elongate filars conjointly wound into respective helical coils having respective windings arranged in an alternating fashion to define a hollow axial lumen. It is mentioned that a flexible hollow tube or porous sponge may be inserted within the axial lumen to serve a reservoir to deliver therapeutic agents. Schaefer et al. note that the bending resistance of the occlusive device may be increased by the inserted tube or sponge.

There are several patents by Greene, Jr. et al., including U.S. Patent No. 7,491,214, disclosing one or more expansile embolizing elements to assist occlusion. These elements may be made of a hydrophilic, macroporous hydrogel foam material. Several types of occlusive devices having a coating material such as a porous, non-absorbable foam, preferably made of polyvinylidene fluoride-co-hexafluropropylene (PVDF/HFP), are disclosed by Yang et al. in U.S. Patent Publication No. 2007/0239205.

US 2006/0058834 discloses an embolization device including an elongated coil which is at least partially embedded in an elongated foam member comprising a flexible, biodegradable, water insoluble, open, interconnecting-cell foam material. The coil may contain reinforcing fiber or fibers that are secured in at least two locations and extend through a lumen of the coil to reduce coil stretching.

It is therefore desirable to have an improved stretch-resistant occlusive device which retains flexibility and conformability during insertion into a vascular malformation yet resists stretching along its entire length when pulling forces are applied to it. It is also desirable to have such a device which enhances thrombus formation and cellular proliferation, especially to treat cerebral aneurysms.

### SUMMARY OF THE INVENTION

Claim 1 defines the invention and the dependent claims disclose the preferred embodiments. An object of the present invention is to maintain high flexibility and conformability in an occlusive device while providing resistance to stretching.

Another object of the present invention is to provide stretch resistance without impairing the ability of an embolic coil to assume a pre-formed shape after delivery to an arterio-venous malformation.

It is yet another object of the invention to provide novel stretch-resistant embolic devices which promote thrombus formation and encourage cellular proliferation to accelerate vessel remodelling within an aneurysm.

This invention results from the realization that stretch resistance can be added to occlusive devices such as embolic coils by utilizing at least one of a stretch-resistant tube and/or one or more filaments together with an elongated porous elastomeric structure which enhances thrombus formation and cellular proliferation.

This invention features an occlusive device as claimed hereinafter.

### BRIEF DESCRIPTION OF THE DRAWINGS

In what follows, preferred embodiments of the invention are explained in more detail with reference to the drawings, in which:
FIG. 1 is a schematic perspective view of a portion of an occlusive device having an elongated porous structure within a helically wound coil;
FIG. 2 is a schematic view of another occlusive device according to the present invention having an elongated porous structure within a stretch-resistant tube;
FIG. 3 is a partially sectioned top view of a vascular occlusive coil hydraulic deployment system with an improved occlusive device according to the present invention;
FIG. 4 is an enlarged partially sectioned view showing the distal gripper portion of the deployment system releasably holding the headpiece of the occlusive device;
FIG. 5 is a schematic rendering of the occlusive device of FIG. 4 being delivered into an aneurysm of a patient;
FIG. 6 is a side sectioned view of the occlusive device shown in FIG. 4;
FIG. 7 is a cross-sectional view of FIG. 6;
FIG. 8 is a side view of another headpiece utilized according to the present invention; and
FIG. 9 is a side view of an anchor filament utilized according to the present invention.

### DETAILED DESCRIPTION OF THE PRESENTLY PREFERRED EMBODIMENTS

Stretch resistance and the ability to enhance thrombus formation is provided to occlusive devices such as embolic coils according to the present invention by utilizing an elongated, substantially cylindrical porous elastomeric structure and a stretch resistant member comprising at least one of an elongated stretch-resistant tube and/or at least one stretch-resistant filament. The elongated porous structure lies within an elongated outer embolic structure, which in some constructions is a helically wound embolic coil.

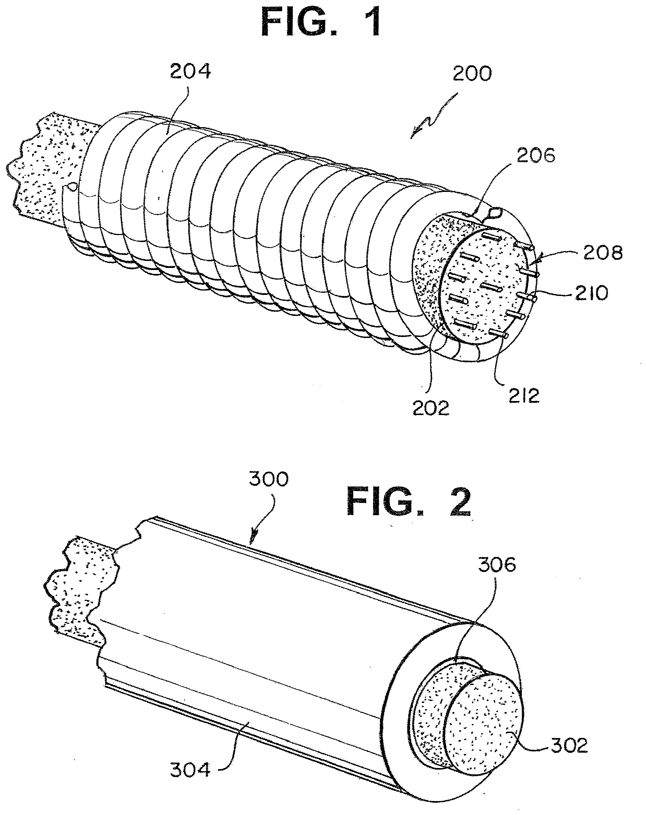

Occlusive device 200, FIG. 1, includes an elongated porous structure 202 within a helically wound coil 204. In this construction, the outer diameter of porous structure 202 is less than the inner diameter of coil 204 such that a gap 206 separates the two components. In this construction, gap 206 decouples porous structure 202 from coil 204 to maintain the flexibility of coil 204. Device 200 further includes stretch resistant member 208 having a plurality of stretch resistant filaments such as filaments 210 and 212.

Preferably, porous structure 202 serves as a three-dimensional elastomeric scaffold for cellular proliferation to accelerate vessel remodelling at the site of an aneurysm into which device 200 is implanted. The average pore size and number of pores affects the overall porosity of structure 202. In some constructions, acceptable parameters include average pore diameters ranging between one micron to 100 microns, more preferably three microns to 50 microns, as measured from scanning electron microscope images along a plane substantially parallel to the surface of the porous structure. In some constructions, the pore sizes are distributed over a wide size range. It is preferred to have a number of pores larger than three microns to easily accommodate platelets to enhance thrombus formation. Suitable biodegradable materials for structure 202 include polycaprolactone-co-glycolyde (Cap-Gly), polyvinylidene fluoride-co-hexafluoropropylene (PVDF-HFP), polyglycolic acid (PGA), and polyglycolic lactic acid (PGLA), also known as poly(lactic-co-glycolic acid)(PLGA). Suitable biocompatible but non-biodegradable materials include urethane, nylon and other polyamides, and expanded polytetrafluoroethylene (EPTFE).

Stretch resistant member 208 has filaments mechanically attached at the terminal ends of embolic coil 204. The cumulative cross-sections of all of the filaments comprising stretch resistant member 208 is sufficient to sustain a desired tensile load, while maintaining overall desired flexibility of device 200. Stretch resistant member 208 can be formed from biocompatible metallic or polymeric materials capable of being manufactured to sustain the desired tensile load. Suitable materials include platinum-tungsten (PtW), stainless steel, gold (Au), platinum-iridium (Ptlr), nylon, Cap-Gly, PGLA, polylactic acid (PLA), EPTFE, polytetrafluoroethylene (PTFE), ethylene tetrafluoroethylene (ETFE), polypropylene, polyester, and nitinol (NiTi). In some constructions, the diameters of stretch-resistant filaments formed from these materials range from 0.0127 mm (0.0005 inches) to 0.076 mm (0.003 inches).

One technique for forming device 200 includes selecting five filaments more than twice as long as the desired final length of device 200 and folding them in half to form proximal bights with ten filament segments running the desired length of device 200. The proximal bights may be slidably engaged with an anchor filament or other proximal retaining element as described below in more detail. Foam is cast about the filaments to form a three-dimensional, open-cell scaffold structure. The distal ends of the filament segments can be melted to form a distal bead as described in more detail below.

In another manufacturing technique, the porous structure is formed as a solidified foam, with internal stretch resistant filaments distributed therein, in an extended length which is then cut to multiple desired lengths, one length for each device according to the present invention. The ends of each foam length is then dissolved to expose the filaments for attachment at one or both ends of a device 200.

In yet another technique, foam material is injected in liquid form into the lumen of a coil containing one or more stretch resistant members, such as coils disclosed by Wilson et al. in U.S. Patent No. 7,572,246, The proximal bight of the stretch resistant member is held by a connector fiber which detachably mounts the stretch resistant member to a pusher member during implantation. Alternatively, foam material is cast around one or more stretch resistant members before the assembly is inserted into the coil lumen.

An alternative occlusive device 300, FIG. 2, includes an elongated porous structure 302 and a stretch resistant tube 304. Porous structure 302 further includes an optional layer 306 which in some constructions is a lubricant and in other constructions is a coating or tube formed of a lubricious or dissolvable material. Preferably, tube 304 is formed of a biodegradable material such as Cap-Gly which degrades relatively quickly to expose structure 302 to blood to enhance thrombus formation. In other constructions, one or more stretch resistant filaments are laid through layer 306 to assist or replace stretch resistance provided by tube 304.

In some constructions, helically wound wire or other materials are wrapped or otherwise placed around tube 304. Although no stretch resistant filaments are illustrated in FIG. 2, one or more filaments can be added within porous structure 302 to enhance the stretch resistance provided by tube 304.

In certain constructions, the occlusive device utilizes a novel proximal headpiece portion as described by Lorenzo et al. in U.S. Patent 2011313443 filed June 16, 2010, entitled "Occlusive Device with Stretch Resistant Member and Anchor Filament". The headpiece defines a headpiece lumen, and a novel proximal anchor filament is passed distally through the headpiece lumen and joined with a flexible distal stretch resistant member. Together, the anchor filament and the stretch resistant member may be referred to as a stretch-resistant assembly which extends along the entire axial interior of the helically wound coil to minimize undue coil elongation without impairing coil flexibility and conformability during and after implantation.

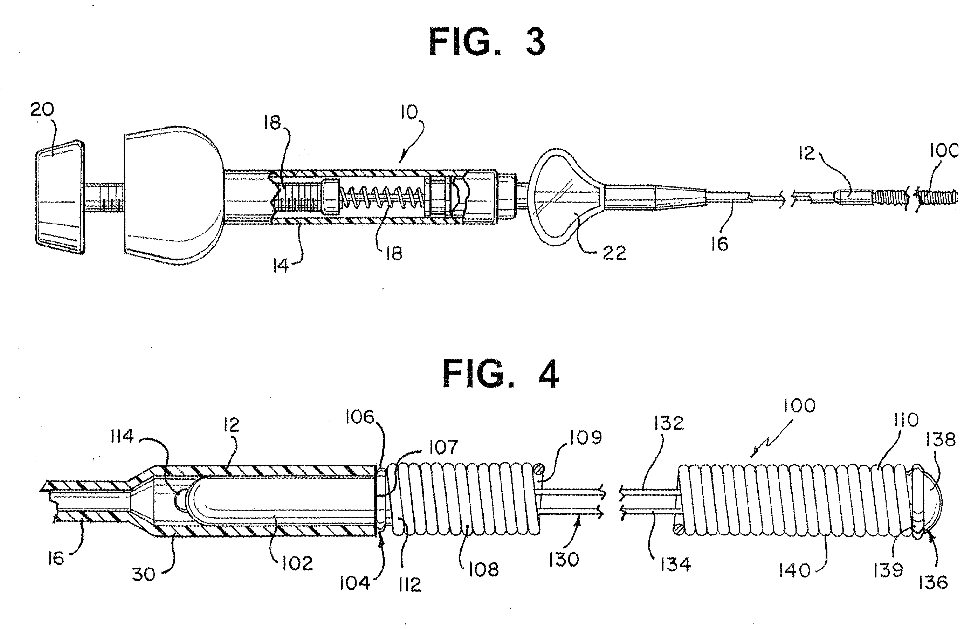

FIG. 3 illustrates an occlusive device 100 according to the present invention releasably held at the distal end 12 of a vascular occlusive coil hydraulic deployment system 10. System 10 includes a hydraulic injector or syringe 14 coupled to the proximal end of a catheter 16. Syringe 14 includes a threaded piston 18 which is controlled by a handle 20 to infuse fluid under high pressure into the interior of catheter 16 when it is appropriate to hydraulically release device 100. Winged hub 22 aids in the insertion of the catheter 104 into the vasculature of a patient.

The occlusive device 100, which is an embolic coil in this construction, and the distal end 12 of catheter 16 are shown in more detail in FIG. 4. For ease of illustration, no foam or other porous structure is shown in FIGS. 4-7. Distal end 12 includes a gripper portion 30, shown in sectional view, tightly holding proximal portion 102 of headpiece 104. Enlarged ring 106 of headpiece 104 limits insertion of headpiece 104 into gripper portion 30, serving as a distal stop as device 100 is releasably connected to catheter 16 prior to insertion into a patient. Another surface of ring 106 serves as a proximal stop during insertion of headpiece distal end 107 into proximal portion 108 of helically wound coil 110, defining proximal coil lumen 109, as described in more detail below. In this construction, proximal-most coil turn 112 of coil 110 abuts ring 106.

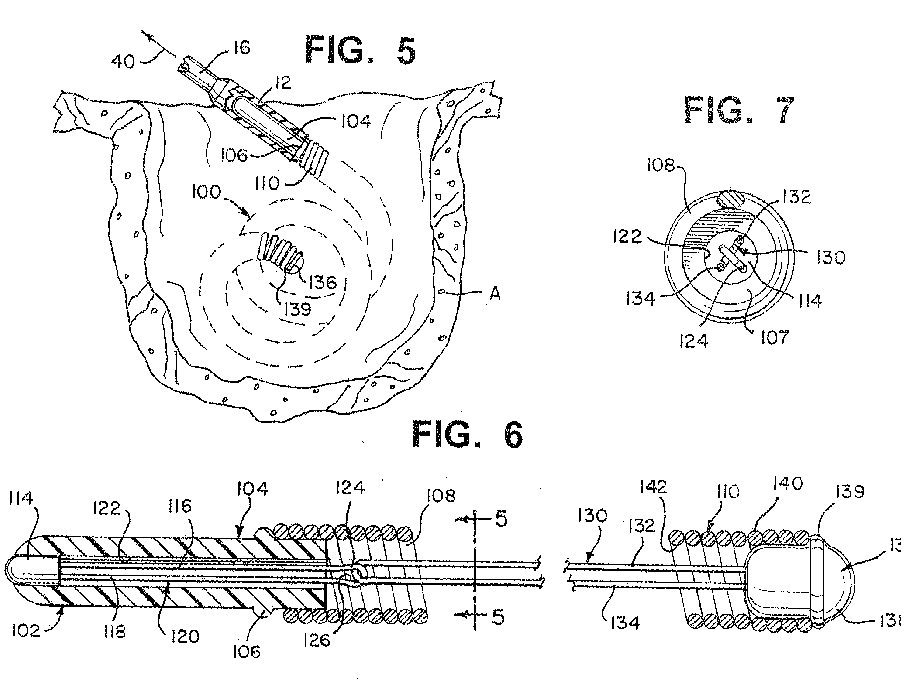

As shown in FIG. 5, distal end 12 of catheter 16 may be retracted in the direction of arrow 40 to reposition embolic coil 100 relative to aneurysm A. When the physician is satisfied with the placement of the entire length of device 100 including its proximal and distal ends, hydraulic pressure is applied, typically at least 10.34 bar (150 psi) to about 48.26 bar (700 psi), more typically between 34.47 bar (500 psi) to 44.81 bar (650 psi), through catheter 16 to forcibly release headpiece 104 and thus relinquish control over device 100. After the headpiece 104 is released, catheter 16 of the delivery system is withdrawn, such as in the direction of arrow 40.

Referring particularly to FIGS. 4 and 6, hydraulic integrity of headpiece 104, which would otherwise be compromised by headpiece lumen 122, necessary to withstand high fluid release pressures is provided in this construction by proximal bead 114 which also structurally secures proximal legs 116 and 118 of anchor filament 120 extending distally through headpiece lumen 122. A distal portion of anchor filament 120 forms bight 124 which defines eye 126. Legs 116 and 118 in this construction are portions of a continuous wire which is folded to form bight 124; in other constructions, anchor filament 120 is a unitary element defining eye 126 similar to a sewing needle having an eye through which thread is passed.

A stretch resistant member 130 passes through eye 126 and extends distally as a loop with two legs 132 and 134 that terminate in distal bead 136 having atraumatic distal surface 138. A cross-sectional view through proximal coil portion 108 showing headpiece distal end 107, and anchor bight 124 distal to headpiece lumen 122, as seen within coil lumen 109 is illustrated in FIG. 7, as if stretch member legs 132 and 134 and coil 110 are extending out of the drawing toward the viewer. Distal bead 136, FIG. 6, is secured to distal portion 140 of coil 110 at least by having an enlarged head 139 which is greater in diameter than distal coil lumen 142 of distal coil portion 140.

A procedure for manufacturing stretch-resistant occlusive devices such as embolic coils includes some or all of the following steps. A distal end of a headpiece is attached to a proximal end portion of a helically wound coil defining a coil lumen extending along the entire axial length of the coil and having a coil distal end portion. The headpiece also has a proximal end and defines a headpiece lumen extending between the proximal and distal ends of the headpiece. Next, a distal portion of an anchor filament, defining an eye, is advanced distally through the headpiece lumen and through the coil lumen to expose the eye beyond the coil distal end portion. A stretch resistant member is passed through the eye to join the member with the filament to create a stretch-resistant assembly extending through the coil lumen and the headpiece lumen, and the anchor filament is retracted to bring the eye in proximity to the distal end of the headpiece. The anchor filament is secured to the proximal end of the headpiece so that the eye is positioned distal to the distal end of the headpiece, and the stretch resistant member is secured to the distal end of the coil, with an atraumatic distal surface, so that proximal and distal ends of the stretch-resistant assembly are secured to resist pulling forces which may be applied to the helically wound coil during implantation in a patient.

Helically wound coil stock is formed initially by winding a platinum-tungsten alloy wire about an elongated, non-curved mandrel to generate tight uniform helical turns defining a central lumen occupied by the mandrel. It is currently preferred for tungsten to comprise approximately six percent to ten percent of the alloy wire. Stiffer framing coils are formed by using round wire having a diameter of approximately 0.076 mm (0.003 inches). More flexible fill coils utilize round alloy wire having a diameter of approximately 0.051 mm (0.002 inches) while even softer coil wire is approximately 0.038 mm (0.0015 inches) in diameter. The softer wire typically is wound over a slightly larger mandrel to generate a slightly larger wound coil diameter defining a correspondingly larger coil lumen. In other constructions, different alloys or material, or a tapered mandrel geometry, could be utilized to alter flexibility of the resulting helically wound coil.

After the mandrel is removed, the initial linear coil stock is cut to desired lengths, typically 1.5 cm to 30 cm, and each length may be thermally "set" into a desired overall curved, non-linear configuration that it will tend to assume after implantation. Configurations having a curved longitudinal axis include a helical or spiral shape and even more complex shapes. Various detachable embolic coils, each having a solid proximal headpiece that is releasably held by a polymeric distal gripper portion of a hydraulic delivery tube during cranial implantation, are currently commercially available as part of the TRUFILL® DCS ORBIT® Detachable Coil System from Codman & Shurtleff, Inc. of Raynham, Massachusetts.

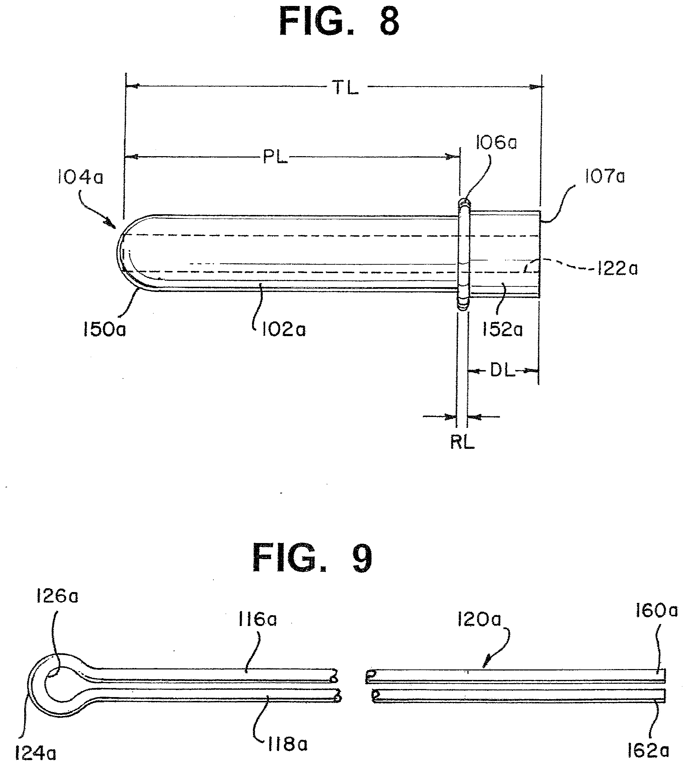

Novel headpieces according to the present invention define a headpiece lumen through which novel anchor filament is passed after the headpiece is attached to the proximal end of the external coil by a solder joint, welding or other secure bond. Examples of compatible headpiece and anchor filament components prior to assemblage are shown in FIGS. 8 and 9, respectively, but are not drawn to scale. Headpiece 104a, FIG. 8, has a total length TL of 1.06 mm (0.042 inches), a proximal portion length PL of 0.86 mm (0.034 inches), and a distal portion length DL of 0.18 mm (0.007 inches). Headpiece 104a defines a headpiece lumen 122a, shown in phantom, having a diameter of approximately 0.1 mm (0.004 inches) and extending from distal end 107a to proximal end 150a. A ring 106a has a longitudinal length RL of 0.025 mm (0.001 inches) and an overall diameter of 0.04 mm (0.0015 inches). Ring 106a separates proximal portion 102a, having a mean proximal diameter of 0.2 mm (0.008 inches), from distal portion 152a, having a mean distal diameter of 0.23 mm (0.009 inches). In other constructions, the mean proximal and distal diameters are substantially the same, as shown in FIG. 6, or may differ by a larger amount, depending on the expected lumen diameters of a delivery catheter gripper portion and a proximal coil lumen, respectively, to be matched with the different portions of the headpiece. Preferably, proximal end 150a, FIG. 8, is curved or chamfered to facilitate mating with the delivery catheter gripper portion, and headpiece 104a is formed of substantially the same material as the helically wound coil to which it will be attached by a secure bond as described above.

Anchor filament 120a, FIG. 9, is formed in this construction using round platinum-tungsten alloy wire, preferably substantially the same alloy as utilized for the headpiece and helically wound coil, having a diameter of approximately 0.04 mm (0.0015 inches), and a length more than twice as great as the combined length of the helically wound coil with attached proximal headpiece. The alloy wire is bent approximately in half, that is, it is doubled over, to form a wire loop having a bight 124a defining an eye 126a with two wire legs 116a and 118a extending in parallel from the bight 124a such as shown in FIG. 9, with an effective length that is greater than the total combined length of the coil and headpiece. The coil with attached headpiece is placed in a first, anchor filament advancement fixture to apply force to both ends until the longitudinal axis becomes substantially non-curved. The anchor bight is advanced distally, through the headpiece lumen and central coil lumen, by pushing on ends 160a and 162a or by grasping the wire legs 116a and 118a of anchor filament 120a, until the bight 124a emerges beyond the distal end of the helically wound coil. After the anchor bight 124a is exposed, a stretch resistant member is threaded through the eye to form a loop extending distally away from the coil.

Sutures provide acceptable stretch resistant members. A preferred non-absorbable suture is PROLENE® polypropylene monofilament suture, especially size 10-0 which is thinner than a human hair, available from Ethicon, Inc. Preferred absorbable sutures include VICRYL® polyglycolic acid monofilament or multifilament sutures, also available from Ethicon, Inc. Other polymeric or metallic fibres or wires can be utilized as desired according to the present invention. Further, the material utilized for the stretch resistant member, or an additive to that material, may be selected to have thrombogenic properties to promote clotting.

Next, the anchor filament with joined stretch resistant member is pulled proximally until the bight is positioned to be spaced several coil wire diameters from the distal end of the headpiece such as shown in FIG. 6. This bight alignment step can be accomplished at the first, anchor filament advancement fixture to maintain a substantially linear longitudinal coil axis, or at another fixture at a subsequent manufacturing station to straighten the helically wound coil during this step. Proper alignment is determined visually in one procedure according to the present disclosure by counting between one to six coil turns extending distally from the headpiece, and positioning the bight within that range of coil turns. It is preferred that the bight does not contact any edges of the headpiece, thereby avoiding potential chafing against the bight or the stretch resistant member. One advantage of the present disclosure is that axial adjustment of the bight during this step of manufacture is readily accomplished by pulling the anchor filament proximally or pulling the stretch resistant member distally to change the position of the bight relative to the headpiece.

After the bight is properly positioned relative to the distal end of the headpiece, excess anchor filament material is trimmed. Heat, such as a plasma flame if the anchor filament is metallic, is applied to the remaining proximal filament ends until they melt and a proximal bead is formed at the proximal end of the headpiece, extending into the headpiece lumen such as shown in FIG. 6, to secure the anchor filament to the headpiece with structural integrity as soon as the bead solidifies. Preferably, the proximal bead appears flush with or smaller than the outer diameter of the headpiece. The solidified proximal bead also restores hydraulic integrity to the headpiece by sealing the headpiece lumen.

Excess stretch resistant member material extending beyond the distal end of the coil is then trimmed, and heat is applied to melt the ends of the remaining material to form a distal bead, preferably concentric and substantially hemispherical in shape with a substantially smooth, atraumatic, low-friction outer surface to facilitate entry and conformance of the occlusive device during its delivery into a malformation of a patient. The amount of stretching of the helically wound coil permitted by the stretch resistant member depends on factors including the composition and thickness of stretch resistant member material, including its tensile properties, as well as the overall length of the stretch resistant member. For example, any desired amount of slack relative to the length of the coil can be established during manufacture by elongating the coil by the desired amount using a fixture before melting the distal portion of the stretch resistant member to form the distal bead, which generates that amount of slack in the stretch resistant member when the coil is released from the fixture.

The anchor filament and stretch resistant member together form a stretch-resistant assembly extending through the coil lumen and the headpiece lumen to minimize coil elongation when pulling forces are applied to the occlusive device. It is desirable for the stretch-resistant assembly to have a pull strength of at least 0.009 kg (0.02 pounds) at its proximal and distal ends when the pull strength of the coil to headpiece solder joint is about 0.023 kg (0.05 pounds).

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention as defined by the claims. For example, it is expressly intended that all combinations of those elements that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. A stretch-resistant occlusive device (300) insertable into the vasculature of a patient, comprising:
an elongated outer embolic structure;
an elongated, substantially cylindrical porous elastomeric structure (302) lying within the elongated outer embolic structure;
an elongated stretch-resistant tube (304) formed from at least one biodegradable material; and
wherein the elongated outer embolic structure is placed around the stretch-resistant tube.

2. The occlusive device of claim 1, wherein a plurality of stretch-resistant filaments are embedded within the elongated porous structure (302).

3. The occlusive device of claim 1, wherein the elongated porous structure (302) includes a layer of a dissolvable material (306) separating it from the stretch-resistant tube (304).

4. The occlusive device of claim 1, wherein the outer embolic structure includes a helically wound coil.

5. The occlusive device of claim 1:
wherein the outer embolic structure includes a substantially cylindrical helically wound coil defining a coil lumen extending along the entire axial length of the coil, the coil having a distal end portion and a proximal end portion with a proximal coil lumen diameter;
wherein the elongated, substantially cylindrical porous elastomeric structure (302) is disposed within the coil lumen; and
wherein at least one stretch resistant filament is positioned within the coil lumen, embedded within the elongated porous structure (302), and secured to at least the distal end portion of the coil.

6. The occlusive device of claim 5, wherein the stretch resistant filament is composed of polymeric material.

7. The occlusive device of claim 1 or claim 5, wherein the elongated porous structure (302) includes open-cell foam material.

8. The occlusive device of claim 1 or claim 5, wherein the elongated porous structure (302) has an average pore diameter of one micron to one hundred microns.

9. The occlusive device of claim 1 or claim 5, wherein the elongated porous structure (302) includes a biodegradable material.

10. The occlusive device of claim 1 or claim 5, wherein the elongated porous structure (302) includes a low-friction layer (306) separating it from the stretch-resistant tube (304).

## Patentansprüche

1. Dehnungsfeste Okklusionsvorrichtung (300), die in das Gefäßsystem eines Patienten einführbar ist, umfassend:
eine langgestreckte äußere embolische Struktur;
eine langgestreckte, im Wesentlichen zylindrische, poröse elastomere Struktur (302), die innerhalb der langgestreckten äußeren embolische Struktur liegt;
ein langgestrecktes dehnungsfestes Rohr (304), das aus wenigstens einem bioabbaubaren Material besteht; und
wobei die langgestreckte äußere embolische Struktur um das dehnungsfeste Rohr angeordnet ist.

2. Okklusionsvorrichtung gemäß Anspruch 1, wobei in der langgestreckten porösen Struktur (302) eine Vielzahl von dehnungsfesten Filamenten eingebettet ist.

3. Okklusionsvorrichtung gemäß Anspruch 1, wobei die langgestreckte poröse Struktur (302) eine Schicht aus einem auflösbaren Material (306) enthält, die sie von dem dehnungsfesten Rohr (304) trennt.

4. Okklusionsvorrichtung gemäß Anspruch 1, wobei die äußere embolische Struktur eine helikal gewundene Spule enthält.

5. Okklusionsvorrichtung gemäß Anspruch 1,
wobei die äußere embolische Struktur eine im Wesentlichen zylindrische, helikal gewundene Spule enthält, die ein Spulenlumen definiert, das entlang der gesamten axialen Länge der Spule verläuft, wobei die Spule einen distalen Endteil und einen proximalen Endteil mit einem proximalen Spulenlumendurchmesser aufweist;
wobei die langgestreckte, im Wesentlichen zylindrische, poröse elastomere Struktur (302) innerhalb des Spulenlumens angeordnet ist; und
wobei wenigstens ein dehnungsfestes Filament in dem Spulenlumen angeordnet, innerhalb der langgestreckten porösen Struktur (302) eingebettet und an wenigstens dem distalen Endteil der Spule befestigt ist.

6. Okklusionsvorrichtung gemäß Anspruch 5, wobei das dehnungsfeste Filament aus Polymermaterial besteht.

7. Okklusionsvorrichtung gemäß Anspruch 1 oder Anspruch 5, wobei die langgestreckte poröse Struktur (302) offenzelliges Schaumstoffmaterial enthält.

8. Okklusionsvorrichtung gemäß Anspruch 1 oder Anspruch 5, wobei die langgestreckte poröse Struktur (302) einen mittleren Porendurchmesser von einem Mikrometer bis einhundert Mikrometer aufweist.

9. Okklusionsvorrichtung gemäß Anspruch 1 oder Anspruch 5, wobei die langgestreckte poröse Struktur (302) ein bioabbaubares Material enthält.

10. Okklusionsvorrichtung gemäß Anspruch 1 oder Anspruch 5, wobei die langgestreckte poröse Struktur (302) eine reibungsarme Schicht (306) enthält, die sie von dem dehnungsfesten Rohr (304) trennt.

## Revendications

1. Dispositif occlusif résistant à l'étirement (300) pouvant être inséré dans le système vasculaire d'un patient, comprenant :
- une structure embolique extérieure allongée ;
- une structure élastomère poreuse, sensiblement cylindrique et allongée (302), reposant à l'intérieur de la structure embolique extérieure allongée ;
- un tube allongé résistant à l'étirement (304) formé d'au moins un matériau biodégradable ;
et
- la structure embolique extérieure allongée étant placée autour du tube résistant à l'étirement.

2. Dispositif occlusif selon la revendication 1, une pluralité de filaments résistant à l'étirement étant intégrés à l'intérieur de la structure poreuse allongée (302).

3. Dispositif occlusif selon la revendication 1, la structure poreuse allongée (302) comprenant une couche d'un matériau dissoluble (306) la séparant du tube résistant à l'étirement (304).

4. Dispositif occlusif selon la revendication 1, la structure embolique extérieure comprenant une bobine enroulée en hélice.

5. Dispositif occlusif selon la revendication 1,
la structure embolique extérieure comprenant une bobine enroulée en hélice sensiblement cylindrique définissant une lumière de bobine s'étendant sur toute la longueur axiale de la bobine, la bobine ayant une partie d'extrémité distale et une partie d'extrémité proximale avec un diamètre de lumière de bobine proximal ;
la structure élastomère poreuse, sensiblement cylindrique et allongée (302) étant disposée à l'intérieur de la lumière de la bobine ; et
au moins un filament résistant à l'étirement étant positionné à l'intérieur de la lumière de la bobine, intégré à l'intérieur de la structure poreuse allongée (302), et fixé à au moins la partie d'extrémité distale de la bobine.

6. Dispositif occlusif selon la revendication 5, le filament résistant à l'étirement étant composé d'un matériau polymère.

7. Dispositif occlusif selon la revendication 1 ou la revendication 5, la structure poreuse allongée (302) comprenant un matériau en mousse à cellules ouvertes.

8. Dispositif occlusif selon la revendication 1 ou la revendication 5, la structure poreuse allongée (302) ayant un diamètre de pore moyen de un micron à cent microns.

9. Dispositif occlusif selon la revendication 1 ou la revendication 5, la structure poreuse allongée (302) comprenant un matériau biodégradable.

10. Dispositif occlusif selon la revendication 1 ou la revendication 5, la structure poreuse allongée (302) comprenant une couche à faible frottement (306) la séparant du tube résistant à l'étirement (304).
